# EUROPÄISCHE PATENTSCHRIFT

(11) **EP 1 138 203 B1**
(45) Veröffentlichungstag und Bekanntmachung des Hinweises auf die Patenterteilung: **28.09.2005**
(21) Anmeldenummer: 01105202.4
(22) Anmeldetag: 03.03.2001
(51) Int. Cl.: A01N 35/02, A01N 25/30, A61L 2/18, A01N 35/04

(54) **Desinfektionssysteme mit mikrobakterizider Wirksamkeit**
Disinfection systems with microbicidal activity
Systèmes de désinfection ayant une activité antimicrobienne

(30) Priorität: 15.03.2000 DE 10012543
(43) Veröffentlichungstag der Anmeldung: 04.10.2001
(73) Patentinhaber: Bode Chemie GmbH & Co., 22525 Hamburg (DE)
(72) Erfinder: Bloss, Richard, Dr., 25462 Rellingen (DE); Fehling, Thomas, 22305 Hamburg (DE); Knieler, Roland, Dr., 21218 Harmstorf (DE); Link, Monika, 22523 Hamburg (DE)
(74) Vertreter: Dinné, Erlend

(56) Entgegenhaltungen:
- EP-A- 0 251 743
- EP-A- 0 265 825
- WO-A-99/15012
- US-A- 4 661 523
- US-A- 5 891 922

## Beschreibung

Gegenstand der Erfindung ist eine mikrobizide Wirkstoffkombination, welche aus wenigstens einem Aldehyd und wenigstens einem kurzkettigen nichtionischen Tensid besteht. Die Erfindung betrifft ferner eine Anwendungslösung, die diese mikrobizide Wirkstoffkombination enthält. Die erfindungsgemäße mikrobizide Wirkstoffkombination weist insbesondere eine hohe antimikrobielle Wirkung gegen Mykobakterien bei gleichzeitiger viruzider und sporozider Aktivität auf. Auch gegenüber anderen Keimen besitzt die erfindungsgemäße Wirkstoffkombination ein ausgeglichenes Wirkungsspektrum.

Als Desinfektionsmittel werden Stoffe bezeichnet, die zur Desinfektion, d. h. zur Bekämpfung pathogener Mikroorganismen (z. B. Bakterien, Viren, Sporen, Klein- und Schimmelpilze) geeignet sind und zwar im allgemeinen durch Anwendung an der Oberfläche von Haut, Kleidung, Geräten, Räumen, aber auch von Trinkwasser, Nahrungsmitteln, Saatgut (Beizen) und als Bodendesinfektionsmittel.

Besonders am Menschen lokal anzuwendende Desinfektionsmittel, z. B. zur Wunddesinfektion, werden auch als Antiseptika bezeichnet.

Desinfektionsmittel werden definiert als Stoffe oder Stoffgemische, die bei der Anwendung auf Gegenständen oder Oberflächen diese in einen Zustand versetzen, in dem sie keine Infektion mehr verursachen können. Ihre Wirkung muß bakterizid, fungizid, viruzid und sporizid (Sammelbegriff: mikrobizid) sein. Ein Effekt im Sinne der Bakteriostase ist für Desinfektionsmittel unzureichend. Sie sind daher im allgemeinen pantoxisch, d. h. sie entfalten ihre Wirkung gegen alle lebenden Zellen.

Je nach Verwendungszweck teilt man die Desinfektionsmittel ein in solche zur Wäsche-, Flächen-, Instrumenten-, Haut- und Hände- sowie zur Stuhl- und Sputumdesinfektion. Unter dem Begriff "Desinfektionsreiniger" versteht man solche Desinfektionsmittel, die gleichzeitig auch als Reinigungs- und gegebenenfalls Pflegemittel fungieren.

Unter Berücksichtigung der vielfältigen Forderungen, die an Desinfektionsmittel gestellt werden, wie z. B. breites Wirkungsspektrum, kurze Einwirkungszeiten, Hautverträglichkeit, geringe Toxizität, Materialverträglichkeit usw. kommen nur einige Wirkstoff-Typen für den Einsatz in Betracht:
1. Die wichtigste Wirkstoff-Gruppe sind die Aldehyde (Formaldehyd, Glyoxal, Glutaraldehyd). Sie besitzen ein breites Wirkungsspektrum einschließlich Virus-Wirksamkeit und sporizider Wirkung bei Formaldehyd und Glutaraldehyd. Aldehyde können allerdings Reste von Blut und Eiweiß durch chemische Reaktion an den zu desinfizierenden Gegenständen fixieren, so daß diese nach der Desinfektion schwer zu reinigen sind.
2. Phenol-Derivate besitzen eine gute bakterizide Wirkung, sind aber nicht sporizid. Gegenüber fast allen anderen Desinfektionsmittelwirkstoffen haben sie den Vorzug, durch Schmutz verhältnismäßig wenig beeinflußt zu werden. Sie eignen sich daher besonders zur Stuhldesinfektion. Typische Vertreter sind 2-Biphenylol und p-Chlorm-kresol (4-Chlor-3-methylphenol). Phenole sind aber vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.
3. Alkohole zeichnen sich durch schnelle Wirksamkeit aus, allerdings erst bei relativ hohen Konzentrationen von ca. 40-80%.
4. Die quaternären Ammonium-Verbindungen, Kationentenside (Invertseifen) und Amphotenside gehören zur Klasse der Tenside. Sie zeichnen sich durch recht gute Haut- und Materialverträglichkeit sowie Geruchsneutralität aus. Ihr Wirkungsspektrum ist dagegen nur begrenzt. Hierher gehören z. B. Benzalkoniumchlorid, Cetrimoniumbromid, Cetylpyridiniumchlorid (Hexadecylpyridiniumchlorid) und andere.
   Quaternäre Ammoniumverbindungen sind organisch Ammoniumverbindungen mit quaternären Stickstoffatomen. Quaternäre Ammoniumverbindungen mit einem hydrophoben Alkyl-Rest sind biozid; ihr Einsatz ist allerdings aus toxikologischen Gründen rückläufig.
   Quaternäre Ammoniumverbindungen werden durch Umsetzung tertiärer Amine mit Alkylierungsmitteln, wie z. B. Methylchlorid, Benzylchlorid, Dimethylsulfat, Dodecylbromid, aber auch Ethylenoxid hergestellt. In Abhängigkeit von dem eingesetzten tertiären Amin unterscheidet man drei Gruppen, welche sich durch die folgenden Strukturformeln auszeichnen, in denen R¹ = CH₃, R² = C₈₋₁₈ und X = Halogen bedeuten: 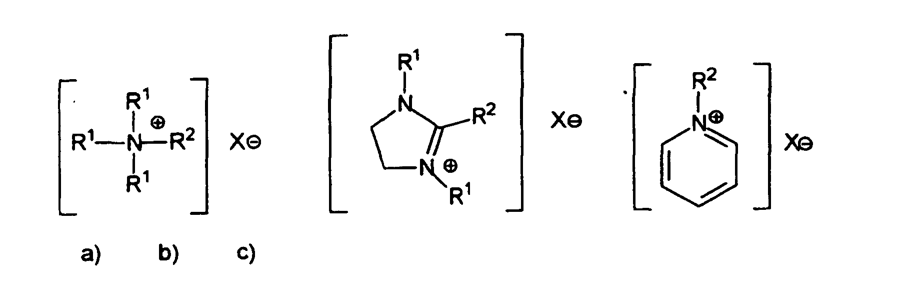
   a) Lineare Alkylammoniumverbindungen
   b) Imidazoliniumverbindungen
   c) Pyridinium-Verbindungen.

   Die Alkylierung tertiärer Amine mit einem langen Alkylrest und zwei Methylgruppen gelingt besonders leicht, auch die Quaternierung tertiärer Amine mit zwei langen Resten und einer Methylgruppe kann mit Hilfe von Methylchlorid unter milden Bedingungen durchgeführt werden. Amine, die über drei lange Alkylreste oder hydroxysubstituierte Alkylreste verfügen, sind wenig reaktiv und werden bevorzugt mit Dimethylsulfat quaterniert.
5. Von den Halogenen besitzen Chlor und lod eine gewisse Bedeutung als Desinfektionsmittel. Chlor ist von der Wasseraufbereitung und Schwimmbaddesinfizierung her bekannt und damit seine unangenehmen Eigenschaften wie Geruch und Korrosivität. Trotz der ausgezeichneten Wirkung gegen Bakterien, Pilze, Sporen und Viren haben chlorhaltige Desinfektionsmittel im Humanbereich aus den obengenannten Gründen und wegen der starken Chlor-Zehrung durch organische Substanzen keine starke Verbreitung gefunden. Dagegen werden Hypochlorite, Chlorkalk- und Chlorisocyanursäuren als technische Desinfektionsmittel noch umfänglich benutzt lodtinktur wird im medizinischen Bereich als Antiseptikum verwendet.
6. Desinfektionsmittel auf Basis von aktivem Sauerstoff (z. B. Wasserstoffperoxid, Peroxyessigsäure) haben in letzter Zeit wieder etwas an Bedeutung gewonnen.

Außer den genannten Mikrobizid-Wirkstoffen sind noch eine Anzahl von mikrobistatischen Substanzen und Konservierungsmitteln (Diphenylether, Carbanilide, Acetanilide aromatischen Säuren und deren Salze) für spezifische Verwendung auf dem Markt, die im erweiterten Sinne den Desinfektionsmitteln zugerechnet werden.

Eine einheitliche Wirkungsweise der Desinfektionsmittel ist nicht zu erkennen. Während manche Präpärate auf die Cytoplasmamembran der Bakterien zerstörend wirken sollen, wird von anderen eine irreversible Blockierung wichtiger Sulfidbindungen bei Enzymen oder von Spurenelementen (durch Chelatisierung) angenommen.

Endoskope sind röhren- oder schlauchförmige Instrumente, welche zur Untersuchung von Körperhohlräumen und Hohlorganen verwendet werden. Sie sind mit einem optischen System, bestehend aus Objektiv und Okular, einer Beleuchtungseinrichtung und meist Spül- und Absaugvorrichtungen sowie Kanälen zum Einführen spezieller Instrumente ausgestattet. Flexible Endoskope (welche auch als Fiberendoskope bezeichnet werden) sind mit einem Glasfaserlichtleiter ausgerüstet, welcher aus einem dicht gepackten Bündel aus haarfeinen und deshalb hochflexiblen Einzelfasern besteht. Durch die Flexibilität der Glasfasern können Licht und Bild über praktisch jede beliebige Abwinklung übertragen werden. Zusätzlich zu den Faserbündeln und den Bowdenzügen für die Bewegung der Spitze sind Kanäle für Wasserspülung und Luft sowie ein in der Regel größerer Biopsie- und gleichzeitig Absaugkanal untergebracht.

Die Endoskopie kann allein, beispielsweise zur Entnahme von Gewebeproben, aber auch in Kombination mit weiteren Methoden, wie z. B. der Ultraschalldiagnostik (Endosonographie) oder der Röntgendiagnostik durchgeführt werden. Gebräuchlich sind beispielsweise Röntgenkontrastdarstellungen der Gallenblase und der Gallengänge sowie des Pankreasgangsystems. Hierbei wird - z. B. im Rahmen einer endoskopischen retrograden Cholangiographie (ERC) bzw. einer endoskopischen retrograden Cholangiopankreatikographie (ERCP) - ein Röntgenkontrastmittel unter Röntgenkontrolle in das zu untersuchende Gangsystem eingebracht.

Wie bereits dargestellt, ist die hohe antimikrobielle Aktivität von Aldehyden bekannt, weshalb sie zur Desinfektion in den weiten Bereichen des Gesundheitswesens, der Industrie etc. eingesetzt werden.

Oft werden allerdings Kombinationen von Aldehyden mit anderen Wirkstoffen, z. B. mit quaternären Ammoniumverbindungen eingesetzt, da Aldehyde ein vergleichsweise hohes allergenes Potential aufweisen. Allerdings gewährleisten solche Kombinationen häufig keine ausreichende Wirkung gegen Mykobakterien (Hygiene & Medizin 1995; 20 Nr. 12, S. 566). Zur Erreichung einer viruziden Wirkung ist nach dem Stand der Technik daher ferner eine Aktivierung durch Zusatz von Alkalien erforderlich.

Ein weiteres Problem stellt das Wirkungsspektrum von Desinfektionsmitteln dar. Auch wenn ein Wirkstoff gegenüber einer Vielzahl von Mikroorganismen in vitro aktiv ist, kann sich dies unter Anwendungsbedingungen aufgrund vieler Beeinflussungen ändern. Insbesondere im Fall der Mycobakterien trifft dies häufig zu, da diese in ihrem Zellwandaufbau zusätzlich durch eine wachsartige Schicht geschützt sind. Aus diesem Grund ist die Zellwand extrem hydrophob und für die meisten mikrobiziden Wirkstoffe schwer zu durchdringen. Dementsprechend oft wird bei der Wirksamkeitsüberprüfung eines Desinfektionsmittels gegen die verschiedenen Keimarten bei Mycobakterien die geringste Wirksamkeit gefunden.

Der Stand der Technik kennt Zubereitungen mit einem Gehalt an Aldehyden, welche zur Desinfektion eingesetzt werden können.

Die US Patente US-3,968,248 und US 3,968,250 beschreiben Zusammensetzungen umfassend wässerige oder alkoholische Glutaraldehydlösungen enthaltend nichtionische und anionische Tenside zur Desinfektion bzw. Sterilisation von medizinischen Instrumenten. EP-A-0 251 743 beschreibt eine flüssige Zusammensetzung zur Sterilisierung umfassend ein nichtonisches Tensid mit 4 bis 12 Ethylenoxideinheiten pro Molekül, Glutaraldehyd und Triethylenglycol.

In DE 689 26 744 T2 werden Zubereitungen beschrieben, die neben Glutaraldehyd unter anderem auch anionische Tenside aus der Gruppe der Alkylsulfate, Alkylsulfonate und Alkylarylsulfonate enthalten und eine gute Wirkung gegen Mykobakterien aufweisen. Nachteilig ist die hohe Neigung der Schaumbildung solcher Mischungen, weshalb die genannten Zubereitungen auch in erster Linie der Vernichtung von Mykobakterien auf der lebenden Oberfläche, d. h. zum Zweck der Hautreinigung dienen.

In der Offenlegungsschrift EP 0 867 115 A2 wird durch den Einsatz großer Mengen eines Ethercarboxylates in Verbindung mit einem geradkettigen oder verzweigten Alkohols eine verbesserte Wirkung speziell bei Mycobakterien erzielt.

In DE 196 36 114 A1 wird offenbart, daß durch Zusatz von Glycolether und/oder Glycolester die Desinfektionswirkung erhöht wird. Um einen Effekt zu erzielen, sind aber auch hier größere Mengen erforderlich, was sich beispielsweise negativ auf die Materialverträglichkeit von Kunststoffen auswirken kann.

Bestimmte nichtionische Tenside sind bekannt für ihre gute Reinigungswirkung bei geringer bis moderater Schaumneigung. Allerdings ist für diese nichtionischen Tenside bisher keine antimikrobielle Aktivität belegt.

Allerdings konnte der Stand der Technik keinerlei Hinweise geben, die den Weg in Richtung der vorliegenden Erfindung gewiesen hätten.

Aufgabe der vorliegenden Erfindung war es, den Nachteilen des Standes der Technik abzuhelfen und ein Desinfektionsmittel auf der Basis von Aldehyden als Wirkstoff zu entwickeln, welches nur eine geringe Schaumentwicklung aufweist. Die Schaumentwicklung sollte bereits bei Raumtemperatur gebremst sein. Eine weitere Aufgabe war, auf den Einsatz biologisch schwer oder nicht abbaubarer Schaumdämpfer, wie z. B. EO-PO Blockpolymere und Silikonderivate zu verzichten. Femer war wesentliche Aufgabe, daß die zu findende Zusammensetzung eine ausgeprägte Wirkung gegen Mycobakterien haben sollte, und zwar im quantitativen Suspensionstest mit Mycobakterium terrae (Hygiene & Medizin 1996; 21 Nr. 7/8, S. 375-380) sowie ferner eine viruzide Wirkung, ohne daß dem Desinfektionsmittel oder seinen Anwendungslösungen Aktivatoren in Form eines nachträglich separat zu dosierenden Produkts, z. B. Natriumhydrogencarbonat zugefügt werden müssen.

Weitere Aufgabe der Erfindung war, daß das Mittel gleichzeitig auch wirksam gegen Bakterien und Pilze sein sollte, und zwar geprüft nach den Richtlinien der DGHM (Deutsche Gesellschaft für Hygiene und Mikrobiologie) gegenüber Staphylococcus aureus, Eschericia coli, Pseudomonas aeruginosa, Proteus mirabills und Candida albicans. Zudem sollte der pH-Wert in der Anwendungslösung nur schwach sauer bzw. neutral eingestellt sein, um die Neigung der Proteinkoagulation des Mittels zu minimieren, eine effektive Reinigung zu ermöglichen und die Verträglichkeit des Mittels gegenüber den üblichen in der Medizin eingesetzten Materialien zu gewährleisten.

Überraschend und für den Fachmann in keiner Weise vorhersehbar werden alle diese Aufgaben gelöst durch ein mikrobizides Wirkstoffkonzentrat, welches in wässriger Lösung
a) 5 bis 30 Gew. -% wenigstens eines aliphatischen, gesättigten Mono- und/oder Dialdehyds enthaltend bis zu 8 Kohlenstoffatome und
b) 0,1 bis 30 Gew. -% wenigstens eines nichtionischen Tensides der Formel

   R- (O-CH₂-CH₂-)ₖ-OH

   worin k eine ganze Zahl von 2 bis 6 und R einen gesättigten oder ungesättigten, linearen oder verzweigten Alkylrest mit 6 bis 10 Kohlenstoffatomen oder einen Phenylalkylrest mit 10 bis 18 Kohlenstoffatomen bedeuten,
enthält, wobei die Gewichtsprozent-Anteile jeweils auf die Gesamtmenge der wäßrigen Lösung bezogen sind.

Die erfindungsgemäßen Wirkstoffkombinationen wirken in ertaunlicher Weise erheblich besser als die Desinfektionsmittel des Standes der Technik. Wirkstoffkombinationen im Sinne der vorliegenden Erfindung sind besonders geeignet zum Desinfizieren von Instrumenten.

Die erfindungsgemäßen Wirkstoffkombinationen können in Form von flüssigen Konzentraten vorliegen und beispielsweise in Dosierbeutel abgepackt werden, so daß eine Gebrauchslösung jeweils frisch angesetzt werden kann. Die flüssigen Konzentrate werden vorzugsweise mit Wasser zu einer Anwendungslösung verdünnt. Bevorzugt sind Gebrauchslösungen, die 0,1 bis 10 Vol.-%, vorzugsweise 0,5 bis 10 Vol.-% an erfindungsgemäßer Wirkstoffkombination enthalten.

Dementsprechend betrifft die vorliegende Erfindung ferner ein mikrobizides Wirkstoffkonzentrat umfassend in wässriger Lösung
(a) 5 bis 30 Gew. -% wenigstens eines aliphatischen, gesättigten Mono- und/oder Dialdehyds enthaltend bis zu 8 Kohlenstoffatome,
(b) 0,1 bis 30 Gew.-% wenigstens eines nichtionischen Tensides der Formel

   R- (O-CH₂-CH₂-)ₖ-OH

   worin k eine ganze Zahl von 2 bis 6 und R einen gesättigten oder ungesättigten, linearen oder vertweigten Alkylrest mit 6 bis 10 Kohlenstoffatomen oder einen Phenylalkylrest mit 10 bis 18 Kohlenstoffatomen bedeuten,
   0,1 bis 40 Gew.-% wenigstens eines weiteren nichtionischen Tensides,
wobei die Gewichts%-Anteile jeweils auf die Gesamtmenge der wäßrigen Lösung bezogen sind.

Die unter (b) aufgeführten Tenside werden im Rahmen dieser Schrift auch als "kurzkettige Tenside" bezeichnet.

Das erfindungsgemäße mikrobizide Wirkstoffkonzentrat besitzt erstaunlicherweise eine sehr gute mikrobizide Wirkung gegen Mykobakterien und Viren, ohne daß die Wirksamkeit gegenüber Bakterien und Pilzen verringert ist.

Das mikrobizide Wirkstoffkonzentrat enthält vorteilhaft insgesamt wenigstens 10 Gew.-% der oben erwähnten Komponenten (a) und (b), bezogen auf die Gesamtmenge des Konzentrats. Der Gehalt an Mono- und/oder Dialdehyden in dem erfindungsgemäßen mikrobiziden Wirkstoffkonzentrat wird vorzugsweise von 5 bis 30 Gew.-%, besonders bevorzugt von 5 bis 25 Gew.-% gewählt, jeweils bezogen auf die Gesamtmenge des Konzentrats.

Bevorzugt werden gesättigte Aldehyde und/oder Dialdehyde mit bis zu 5 Kohlenstoffatomen eingesetzt. Ganz besonders vorteilhaft sind Glutaraldehyd, Formaldehyd, Glyoxal, Succinaldehyd, o-Phthalaldehyd oder deren Mischungen.

Der Gehalt an erfindungsgemäßem kurzkettigen nichtionischen Tensiden in dem erfindungsgemäßen milkrobiziden Wirkstoffkonzentrat wird vorzugsweise von 0,5 bis 20 Gew.-%, besonders bevorzugt von 3 bis 10 Gew.-% gewählt, jeweils bezogen auf die Gesamtmenge des Konzentrats.

Vorzugsweise enthält der Alkylrest R aus der Formel R- (O-CH₂-CH₂-)ₖ-OH 6 bis 10, insbesondere bevorzugt 8 Kohlenstoffatome. Bevorzugt nimmt k eine ganze Zahl zwischen 2 und 6, besonders bevorzugt zwischen 4 und 6 an. Ein bevorzugtes nichtionisches Tensid ist C₈-Alkylethoxylat (4 EO), d. h. der Alkylrest hat 8 Kohlenstoffatome und k = 4.

Der Gehalt an weiteren nichtionischen Tensiden wird vorzugsweise aus einem Bereich von 0,1 bis 30 Gew.-%, besonders bevorzugt von 0,1 bis 20 Gew.-% gewählt, jeweils bezogen auf die Gesamtmenge des Konzentrats. Besonders bevorzugte weitere nichtionische Tenside sind C₁₂₋₁₈-Fettalkoholalkoxylate (alkoxyethoxy- und/oder propoxy-), insbesondere C₁₂₋₁₈-Fettalkoholethoxylate oder Gemische.

Das erfindungsgemäße mikrobizide Wirkstoffkonzentrat kann ferner bis zu 50 Gew.-%, vorzugsweise bis zu 30 Gew.-%, bezogen auf die Gesamtmenge des Konzentrats, eines geradkettigen oder verzweigten gesättigten Alkohols mit bis zu 8 Kohlenstoffatomen enthalten. Bevorzugte Alkohole sind Ethanol, 1 -Propanol und 2- Propanol.

Das erfindungsgemäße mikrobizide Wirkstoffkonzentrat kann als solches oder in Lösung in einem geeigneten Lösungsmittel oder Lösungsmittelgemisch zur Anwendung kommen. Dementsprechend betrifft die vorliegende Erfindung auch eine Anwendungslösung, welche erhältlich ist, indem das oben beschriebene mikrobizide Wirkstoffkonzentrat in einem geeigneten Lösungsmittel oder Lösungsmittelgemisch gelöst wird.

Die erfindungsgemäße Anwendungslösung enthält das Wirkstoffkonzentrat vorteilhaft in einem Anteil von 0,1 bis 10 Vol.-%, vorzugsweise von 0,5 bis 10 Vol.-%, bezogen auf das Gesamtvolumen der Anwendungslösung (Anwendungskonzentration).

Als Lösungsmittel kommen Wasser oder auch ein Wasser/Alkoholgemisch in Frage, wobei als Alkohol vorzugsweise kurzkettige Alkohole mit einer Kettenlänge von 2 bis 3 Kohlenstoffatomen eingesetzt werden, insbesondere Ethanol, 1-Propanol oder 2-Propanol.

Die Anwendungslösung hat vorzugsweise einen pH-Wert zwischen 3,5 und 7, vorzugsweise zwischen 4 und 7, um eine optimale Schonung des empfindlichen Materials zu erreichen.

Die erfindungsgemäßen Wirkstoffkombinationen können in Form von Wirkstoffkonzentraten oder verdünnt als Anwendungslösungen zur Desinfektion oder Sterilisation von unbelebten Oberflächen aller Art verwendet werden, insbesondere zur Desinfektion von chirurgischem Instrumentarium und Anästhesiematerialien, wie starren oder flexiblen Endoskopen. Die Desinfektion oder Sterilisation kann nach allen bekannten Verfahren durch Behandeln der Oberfläche mit der erfindungsgemäßen Wirkstoffkombination erfolgen. Es kann auch vorteilhaft sein, die zu desinfizierenden Gegenstände im Ultraschallbad mit der erfindungsgemäßen Wirkstoffkombinationen zu behandeln.

Erfindungsgemäße mikrobizide Wirkstoffkonzentrate können ferner vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 5 Gew.-%, vorzugsweise bis zu 2 Gew.% bezogen auf das Gesamtgewicht der Formulierung, zu wählen.

Die erfindungsgemäßen Wirkstoffkonzentrate können darüberhinaus vorteilhaft zusätzlich Komplexbildner und/oder pH-Regulatoren enthalten, um den negativen Einfluß schwankender Wasserhärte zu reduzieren bzw. um zu gewährleisten, daß der pH-Wert des reinigenden und desinfizierenden Systems in einem Bereich liegt, der das empfindliche Material nicht schädigt.

Zusätzlich zu den vorstehend genannten Komponenten können die erfindungsgemäßen Wirkstoffkonzentrate für derartige Zubereitungen übliche Farbstoffe, Duftstoffe und/oder andere übliche Hilfsstoffe enthalten. Diese tragen in der Mehrzahl der Fälle nicht zur reinigenden Wirkung bei, sondern dienen der Lagerbarkeit sowie ästhetischen Zwecken. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die vorliegende Erfindung wird durch die nachfolgenden Beispiele näher beschrieben, ohne jedoch darauf beschränkt zu sein.

### Beispiele:

Es wurden vier Rezepturen erstellt. Vergleichsrezepturen V1 und V2 entsprechen typischen im Markt befindlichen Produkten des Standes der Technik, während die Beispielrezepturen B3 und B4 erfindungsgemäße Zubereitungen darstellen.

**Tabelle 1:**

| **Rezepturbestandteil** | **V1** | **V2** | **B3** | **B4** |
|---|---|---|---|---|
| Glutaraldehyd | 10 | 7,5 | 10 | 7,5 |
| Formaldehyd-Abspalter | 11 | 15 | 11 | 15 |
| Didecyldimethylammoniumchlorid | 1 | 1 | 1 | 1 |
| Benzalkoniumchlorid | 1 | 1 | 1 | 1 |
| Glykol | - | 10 | - | 10 |
| Isopropanol | 6 | 6 | 6 | 6 |
| C₈-Alkylethoxylat (4 EO) | - | - | 8 | 8 |
| C₁₃-Fettalkoholethoxylat 7 EO | 4 | 4 | - | - |
| C₁₂₋₁₄-Fettalkoholpolyglykolether 12 EO | 4 | 4 | - | - |
| Korrosionsschutz | 2,5 | 2,5 | 2,5 | 2,5 |
| Wasser | Rest | Rest | Rest | Rest |
| EO: Zahl der Ethoxylatgruppen | | | | |

Der pH-Wert der Rezepturen wurde jeweils in gleicher Weise auf 4,0 mit Natronlauge eingestellt.

Die Rezepturen wurden zunächst auf die üblichen Anwendungskonzentrationen (AWK) verdünnt und diese verdünnten Lösungen dann auf ihre Wirksamkeit gegen Mykobakterien getestet. Die Ergebnisse sind nachfolgender Tabelle 2 zu entnehmen.

**Tabelle 2**

| **Konzentration/Zeit** | **V1** | **V2** | **B3** | **B4** |
|---|---|---|---|---|
| 6%/15min | n.d. | 5,34 | n.d. | 5,34 |
| 6%/30min | n.d. | 5,34 | n.d. | 5,34 |
| 6%/60min | n.d. | 5,26 | n.d. | 5,26 |
| 4%/15min | 3,13 | 3,34 | 6,08 | 5,34 |
| 4%/30min | n.d. | 5,34 | n.d. | 5,34 |
| 4%/60min | n.d. | 5,34 | n.d. | 5,26 |
| 3%/15min | 2,27 | n.d. | 6,08 | n.d. |
| 3%/30min | 6,07 | n.d. | 6,07 | n.d. |
| 2%/15min | 1,08 | 3,04 | 2,63 | 5,34 |
| 2%/30min | 2,22 | 5,34 | 6,07 | 5,34 |
| 2%/60min | n.d. | 5,26 | n.d. | 5,26 |

| | |
|---|---|
| n.d. | nicht durchgeführt |
| Testkeim | Mycobacterium terrae ATCC 15755 |
| KBE log/ml der Prüfsuspension | 9,90 log/ml (Rez. V1 und B3) und 9,08 log/ml (Rez. V2 und B4) |
| Prüftemperatur | 20 ± 1 °C |

Die Ergebnisse sind in log Reduktionsfaktoren angegeben.

Es zeigt sich deutlich, dass die Rezepturen mit dem kurzkettigen Tensid eindeutig bessere mykobakterizide Wirksamkeiten bei gleichem bakteriziden Wirkstoffanteil besitzen.

## Patentansprüche

1. Mikrobizides Wirkstoffkonzentrat, welches in wässriger Lösung
a) 5 bis 30 Gew. -% wenigstens eines aliphatischen, gesättigten Mono- und/oder Dialdehyds enthaltend bis zu 8 Kohlenstoffatome und
b) 0,1 bis 30 Gew. -% wenigstens eines nichtionischen Tensides der Formel
R- (O-CH₂-CH₂-)ₖ-OH
worin k eine ganze Zahl von 2 bis 6 und R einen gesättigten oder ungesättigten, linearen oder verzweigten Alkylrest mit 6 bis 10 Kohlenstoffatomen oder einen Phenylalkylrest mit 10 bis 18 Kohlenstoffatomen bedeuten,
enthält, wobei die Gewichtsprozent-Anteile jeweils auf die Gesamtmenge der wäßrigen Lösung bezogen sind.

2. Wirkstoffkonzentrat nach Anspruch 1, **dadurch gekennzeichnet, daß** es 10 bis 30 Gew.-% wenigstens eines aliphatischen, gesättigten Mono- und/oder Dialdehyds enthaltend bis zu 8 Kohlenstoffatome enthält.

3. Wirkstoffkonzentrat nach Anspruch 1 oder 2, **dadurch gekennzeichnet, daß** zusätzlich weitere Tenside und/oder Hilfs-, Zusatz- und/oder weitere Wirkstoffe und/oder Lösungsmittel enthalten sind.

4. Wirkstoffkonzentrat nach einem der Ansprüche 1 bis 3, **dadurch gekennzeichnet, daß** es zusätzlich einen geradkettigen oder verzweigten Alkohol mit bis zu 8 Kohlenstoffatomen enthält.

5. Wirkstoffkonzentrat nach einem der Ansprüche 1 bis 4, **dadurch gekennzeichnet, daß** das oder die Aldehyde gewählt werden aus der Gruppe Glutaraldehyd, Formaldehyd, Glyoxal, Succinaldehyd, o-Phthalaldehyd oder beliebige Mischungen hiervon.

6. Wirkstoffkonzentrat nach mindestens einem der Ansprüche 1 bis 5, **dadurch gekennzeichnet, daß** der Gehalt an nichtionischen Tensiden (eine oder mehrere Verbindungen) von 0,5 bis 20 Gew.-% gewählt wird, bezogen auf die Gesamtmenge des Konzentrats.

7. Mikrobizides Wirkstoffkonzentrat nach Anspruch 1 **dadurch gekennzeichnet, daß** es
a) 2 bis 10 Gew.-% eines oder mehrerer Alkohole und
b) 1 bis 5 Gew.-% einer oder mehrerer quatemärer Ammoniumverbindungen enthält.

8. Anwendungslösung zur Desinfektion oder Sterilisation von unbelebten Oberflächen, **dadurch** gekeinnzeichnet, daß sie 0,1 bis 10 Vol.-% eines Wirkstoffkonzentrates nach einem der Ansprüche 1 bis 6 enthält, wobei die Lösung **dadurch** erhältlich ist, daß das Wirkstoffkonzentrat mit Wasser oder einem Wasser/Alkoholgemisch verdünnt wird.

9. Verwendung eines Wirkstoffkonzentrats nach einem der Ansprüche 2 bis 6 oder einer Anwendungslösung nach Anspruch 8 zur Desinfektion oder Sterilisation von medizinischen Instrumenten.

## Claims

1. Microbicidal active substance concentrate, which in aqueous solution contains
a) 5 to 30% by weight of at least one aliphatic, saturated mono- and/or dialdehyde comprising up to 8 carbon atoms and
b) 0.1 to 30% by weight of at least one nonionic surfactant of the formula
**R-(O-CH**_{**2**}**-CH**_{**2**}**-)**_{**k**}**-OH**
in which k represents a whole number from 2 to 6 and R a saturated or unsaturated, linear or branched alkyl radical with 6 to 10 carbon atoms or one phenylalkyl radical with 10 to 18 carbon atoms,
whereby the weight percentage contents refer to the total amount of the aqueous solution respectively.

2. Active substance concentrate according to Claim 1, **characterized** thereby that it contains 10 to 30% by weight of at least one aliphatic, saturated mono- and/or dialdehyde comprising up to 8 carbons.

3. Active substance concentrate according to Claim 1 or 2, **characterized** thereby that further surfactants and/or additives and/or further active agents and/or solvents are additionally contained therein.

4. Active substance concentrate according to one of Claims 1 to 3, **characterized** thereby that it additionally contains a straight-chain or branched alcohol with up to 8 carbon atoms.

5. Active substance concentrate according to one of Claims 1 to 4, **characterized** thereby that the aldehyde or aldehydes are chosen from the group glutaraldehyde, formaldehyde, glyoxal, succinaldehyde, o-Phthalaldehyde or any mixtures hereof.

6. Active substance concentrate according to at least one of Claims 1 to 5, **characterized** thereby that the content of nonionic surfactants (one or more compounds) is chosen from 0.5 to 20% by weight, in relation of the total amount of the concentrate.

7. Microbicidal active substance concentrate according to Claim 1, **characterized** thereby that it contains
a) 2 to 10% by weight of one or more alcohols and
b) 1 to 5% by weight of one or more quaternary ammonium compounds.

8. Application solution for the disinfection or sterilization of inanimate surfaces, **characterized** thereby that it contains 0.1 to 10% by volume of an active substance concentrate according to one of Claims 1 to 6, whereby the solution is attained by diluting the active substance concentrate with water or a water/alcohol mixture.

9. Use of an active substance concentrate according to one of Claims 2 to 6 or an application solution according to Claim 8 for the disinfection or sterilization of medical instruments.

## Revendications

1. Concentré de substances microbicides, comprenant en solution aqueuse
a) 5 à 30 % en poids au moins d'un monoaldéhyde et / ou d'un dialdéhyde aliphatique saturé renfermant jusqu'à 8 atomes de carbone et
b) 0,1 à 30 % en poids au moins d'un tensio-actif non ionique de formule
**R-(O-CH**_{**2**}**-CH**_{**2**}**-)**_{**x**}**-OH**
dans laquelle k représente un chiffre compris entre 2 et 6 et où R représente un radical alkyle saturé ou insaturé, à chaîne linéaire ou ramifiée avec 6 à 10 atomes de carbone ou un radical phénylalkyl avec 10 à 18 atomes de carbone, où le pourcentage en poids se rapporte au poids total de la solution aqueuse,
où le pourcentage en poids se rapporte au poids total de la solution aqueuse

2. Concentré de substances actives selon la revendication 1, **caractérisé en ce qu'**il renferme entre 10 et 30 % en poids au moins d'un monoaldéhyde et / ou d'un dialdéhyde aliphatique saturé comprenant jusqu'à 8 atomes de carbone.

3. Concentré de substances actives selon la revendication 1 ou 2, **caractérisé en ce qu'**il renferme également des tensio-actifs et / ou des auxiliaires, des additifs et / ou d'autres substances actives et / ou un solvant.

4. Concentré de substances actives selon l'une quelconque des revendications 1 à 3, **caractérisé en ce qu'**il renferme également un alcool à chaîne droite ou ramifiée comprenant jusqu'à 8 atomes de carbone.

5. Concentré de substances actives selon l'une quelconque des revendications 1 à 4, **caractérisé en ce que** le ou les aldéhyde(s) est ou sont choisi(s) dans le groupe comprenant le glutaraldéhyde, le formaldéhyde, le glyoxal, l'aldéhyde succinique, o-phthalaldéhyde ou tout mélange de ces substances.

6. Concentré de substances actives selon au moins l'une quelconque des revendications 1 à 5, **caractérisé en ce que** la teneur en tensio-actifs non ioniques (une ou plusieurs liaisons) est choisie dans l'intervalle 0,5 à 20 % en poids par rapport au poids total du concentré.

7. Concentré de substances microbicides selon la revendication 1, **caractérisé en ce qu'**il renferme
a) 2 à 10 % en poids d'un ou plusieurs alcool(s) et
b) 1 à 5 % en poids d'une ou plusieurs liaison(s) ammonium(s) quaternaire(s).

8. Solution prête à l'emploi destinée à la désinfection ou à la stérilisation de surfaces non vivantes, **caractérisée en ce qu'**elle renferme 0,1 à 10 % de volume d'un concentré de substances actives selon l'une quelconque des revendications 1 à 6, moyennant quoi la solution est obtenue par dissolution du concentré de substances actives avec de l'eau ou un mélange eau / alcool.

9. Utilisation d'un concentré de substances actives selon l'une quelconque des revendications 2 à 6 ou d'une solution prête à l'emploi selon la revendication 8 pour la désinfection ou la stérilisation d'instruments médicaux.
